(19) Europäisches Patentamt / European Patent Office / Office européen des brevets

(11) **EP 3 972 280 A1**

(12) **EUROPEAN PATENT APPLICATION**
published in accordance with Art. 153(4) EPC

(43) Date of publication:
**23.03.2022 Bulletin 2022/12**

(21) Application number: **21777191.4**

(22) Date of filing: **02.07.2021**

(51) International Patent Classification (IPC):
**H04R 1/00** (2006.01)   **H04R 1/02** (2006.01)
**H04R 1/28** (2006.01)   **G10K 11/24** (2006.01)
**A47C 27/00** (2006.01)

(52) Cooperative Patent Classification (CPC):
**A47C 7/40; A47C 7/62; A47C 7/72; A47C 27/00; A61M 21/02; G10K 11/02; G10K 11/24; H04R 1/00; H04R 1/02; H04R 1/20; H04R 1/28; H04R 7/02**

(86) International application number:
**PCT/JP2021/025150**

(87) International publication number:
**WO 2022/019091 (27.01.2022 Gazette 2022/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.07.2020 JP 2020123595**

(71) Applicant: **AIN KOUSAN CO., LTD.**
**Shinagawa-ku,**
**Tokyo 1410031 (JP)**

(72) Inventor: **NISHIBORI, Sadao**
**Shinagawa-ku, Tokyo 1410031 (JP)**

(74) Representative: **Betten & Resch**
**Patent- und Rechtsanwälte PartGmbB**
**Maximiliansplatz 14**
**80333 München (DE)**

(54) **ACOUSTIC DEVICE, AND ACOUSTIC SYSTEM**

(57)   An acoustic apparatus (2) includes: a three-dimensional network structural body (7) that is an aggregate of filaments (70) that are made of a resilient resin, each randomly looped or curled, fused to each other, and intertwined with each other; an at least one speaker (5) that generates acoustic waves; and a board-like resonance board (6). The three-dimensional network structural body (7) has a front surface (73) that is held in direct or indirect contact with a human body, and an inner surface (74) that faces the front surface (73). The at least one speaker (5) includes a vibrating portion (51) that faces the inner surface (74) of the three-dimensional network structural body (7), and a frame portion (52) that holds the vibrating portion (51) while allowing the vibrating portion (51) to vibrate. The frame portion (52) is mounted to one side surface (611) of the board-like resonance board (6) through intermediation of a resilient mounting material (8).

【FIG.3A】

【FIG.3B】

**Description**

Technical Field

The present invention relates to an acoustic apparatus and an acoustic system.

Background Art

Acoustic systems including speakers incorporated in a cushion of a seat or the like have been known. Patent Literature 1 cited below discloses an acoustic system configured to transmit acoustic waves of the speakers to the back side of the upper half of a human body via a three-dimensional network structural body formed of randomly curved or looped filaments of a thermoplastic resin.

Citation List

Patent Literature

[PTL 1] Japan Patent No. 4907991

Summary of Invention

Technical Problem

In the acoustic system disclosed in Patent Literature 1, the acoustic waves from the speakers are transmitted to the human body (especially to the back) via the filaments forming the three-dimensional network structural body. With this, for example, a state as if vibrations of strings of a musical instrument are transmitted directly to the human body occurs. In this way, physical and mental relaxation effect that is deeper than that at a time when sound is perceived only through air can be obtained.

However, the acoustic waves generated by the speakers are propagated not only to the three-dimensional network structural body but also, for example, to a casing or a housing to which the speakers are mounted. If how the acoustic waves are propagated by such members other than the three-dimensional network structural body is not taken into account in the acoustic system disclosed in Patent Literature 1, the acoustic waves are liable to be propagated to parts other than the back by these members, and the sound is liable to be propagated through the air. As a result, the above-mentioned relaxation effect to be obtained from the propagation of the acoustic waves to the human body (specifically, to the back) via the filaments of the three-dimensional structural body is liable to be degraded.

The present invention has been made in view of such circumstances, and an object thereof is to provide an acoustic apparatus and an acoustic system that enable acoustic waves to be effectively propagated to a human body via filaments of a three-dimensional network structural body.

Solution to Problem

According to a first aspect of the present invention, there is provided an acoustic apparatus that transmits acoustic waves to a human body, the acoustic apparatus including:

a three-dimensional network structural body that is an aggregate of filaments that are

made of a resilient resin,
each randomly looped or curled,
fused to each other, and
intertwined with each other;

a plurality of speakers that generate the acoustic waves; and
a board-like resonance board,
in which the three-dimensional network structural body has

a front surface that is held in direct or indirect contact with the human body, and
an inner surface that faces the front surface,

in which the plurality of speakers respectively include

vibrating portions that face the inner surface of the three-dimensional network structural body, and
frame portions that respectively hold the vibrating portions while allowing the vibrating portions to vibrate,

in which the frame portions are mounted to one side surface of the board-like resonance board through intermediation of a resilient mounting material,
in which the board-like resonance board includes

a plurality of wooden boards that correspond one on one to the plurality of speakers, and
a board member to which the plurality of wooden boards are mounted,

in which the frame portions of the plurality of speakers are mounted respectively to one side surfaces of corresponding ones of the plurality of wooden boards through intermediation of the resilient mounting material, and
in which the plurality of wooden boards mounted respectively to the frame portions each have another side surface that faces the one side surface of a corresponding one of the plurality of wooden boards, and that is mounted to one side surface of the board member through intermediation of the resilient mounting material.

Preferably,

the frame portions each have a circular-flat bottom surface,
a recessed portion is formed at a center of the circular-flat bottom surface,
the resilient mounting material includes

one part applied in the recessed portion, and
another part applied at least to a part of an outer rim portion of the circular-flat bottom surface, and

the frame portions are mounted respectively to the one side surfaces of the corresponding ones of the plurality of wooden boards through intermediation of the one part and the other part of the resilient mounting material.

Preferably, another acoustic apparatus transmits acoustic waves to a human body, the other acoustic apparatus including:

a three-dimensional network structural body that is an aggregate of filaments that are

made of a resilient resin,
each randomly looped or curled,
fused to each other, and
intertwined with each other;

an at least one speaker that generates the acoustic waves; and
a board-like resonance board,
the three-dimensional network structural body has

a front surface that is held in direct or indirect contact with the human body, and
an inner surface that faces the front surface,

the at least one speaker includes

a vibrating portion that faces the inner surface of the three-dimensional network structural body, and
a frame portion that holds the vibrating portion while allowing the vibrating portion to vibrate,

the frame portion is mounted to one side surface of the board-like resonance board through intermediation of a resilient mounting material,
the board-like resonance board includes

a wooden board and
a metal board,

the metal board covers at least a part of one side surface of the wooden board, and
the frame portion is mounted to one side surface of the metal board through intermediation of the resilient mounting material.

Preferably,

the frame portion has a circular-flat bottom surface,
a recessed portion is formed at a center of the circular-flat bottom surface,
the resilient mounting material includes

one part applied in the recessed portion, and
another part applied at least to a part of an outer rim portion of the circular-flat bottom surface, and

the frame portion is mounted to the one side surface of the metal board through intermediation of the one part and the other part of the resilient mounting material.

Preferably,
the metal board mounted to the frame portion has another side surface that faces the one side surface of the metal board, and that is mounted to the one side surface of the wooden board through intermediation of the resilient mounting material.

Preferably,

the resilient mounting material includes parts applied to a plurality of points away from each other on the other side surface of the metal board, and
the other side surface of the metal board is mounted to the one side surface of the wooden board through intermediation of the parts of the resilient mounting material.

Preferably,

the at least one speaker includes a plurality of speakers,
the wooden board of the board-like resonance board includes a plurality of wooden boards that correspond one on one to the plurality of speakers,
the board-like resonance board includes a board member to which the plurality of wooden boards are mounted,
one wooden board of the plurality of wooden boards and the frame portion of one speaker of the plurality of speakers,
the one speaker corresponding to the one wooden board, are mounted to both the one side surface and the other side surface of the metal board in a manner that the one speaker and the one wooden board face each other across the metal board, and
the other side surface of the one wooden board mounted to the metal board
faces the one side surface of the one wooden board, and
is mounted to one side surface of the board member through intermediation of the resilient mounting material.

Preferably,
the metal board is a copper board.
Preferably,

the three-dimensional network structural body has

a rear surface

that faces the front surface, and
that is farther from the front surface than from the inner surface, and

inner side surfaces that surround the speaker between the inner surface and the rear surface, and

the speaker is housed in an interior space surrounded

by the inner surface and the inner side surfaces of the three-dimensional network structural body, and
by the one side surface of the board-like resonance board.

Preferably,
the rear surface of the three-dimensional network structural body is mounted to the one side surface of the board-like resonance board through intermediation of the resilient mounting material.

Preferably,
the resilient mounting material is

a thermoplastic elastomer including ethylene-vinyl acetate copolymer, or
glue.

According to the present invention, there is provided an acoustic system, including:

a body portion having a support surface that supports at least the back of a human body; and
a board-like base member on which the body portion is placed,
in which the body portion includes

the above-described acoustic apparatus that transmits the acoustic waves to the human body through the support surface, and
a holding portion that holds the above-described acoustic apparatus, and

in which the base member includes

a first board member,
a second board member that is arranged under the first board member, and
a resilient buffer member that is inserted between the first board member and the second board member.

Preferably,
the first board member is a wooden board member.

Preferably,

the resilient buffer member is made of

glass wool or
rock wool.

Preferably,
the second board member includes

a wooden upper-board member that faces the resilient buffer member,
a wooden lower-board member that is arranged under the wooden upper-board member, and
an elastomeric sheet that is inserted between the wooden upper-board member and the wooden lower-board member.

Preferably,
the holding portion includes a resilient member that holds the above-described acoustic apparatus.

In addition, according to the present invention, there is provided a still another acoustic measure that transmits acoustic waves to a human body, the still other acoustic measure including:

a three-dimensional network structural body that is an aggregate of filaments that are

made of a resilient resin,
each randomly looped or curled,
fused to each other, and
intertwined with each other;

an at least one speaker that generates the acoustic waves; and
a board-like resonance board,
in which the three-dimensional network structural body has

a front surface that is held in direct or indirect contact with the human body, and
an inner surface that faces the front surface, in which the at least one speaker includes
a vibrating portion that faces the inner surface of the three-dimensional network structural body, and
a frame portion that holds the vibrating portion while allowing the vibrating portion to vibrate, and

in which the frame portion is mounted to one side surface of the board-like resonance board through intermediation of a resilient mounting material.

Preferably,

the board-like resonance board includes a wooden board, and
the frame portion is mounted to one side surface of the wooden board through intermediation of the resilient mounting material.

Preferably,

the frame portion has a circular-flat bottom surface,
a recessed portion is formed at a center of the circular-flat bottom surface,
in which the resilient mounting material includes

one part applied in the recessed portion, and
another part applied at least to a part of an outer rim portion of the circular-flat bottom surface, and

the frame portion is mounted to the one side surface of the wooden board through intermediation of the one part and the other part of the resilient mounting material.

Advantageous Effects of Invention

According to the present invention, it is possible to provide the acoustic apparatus and the acoustic system that enable the acoustic waves to be effectively propagated to a human body via the filaments of the three-dimensional network structural body.

Brief Description of Drawings

[FIG. 1] FIG. 1 is a view illustrating an example of a configuration of an acoustic system according to an embodiment of the present invention.
[FIG. 2] FIG. 2 is a view illustrating a state in which an acoustic apparatus is removed from a body portion in the acoustic system illustrated in FIG. 1.
[FIG. 3] FIG. 3A is a view illustrating an example of an external appearance of the acoustic apparatus according to the embodiment, and FIG. 3B is a cross-sectional view illustrating an example of a structure of the acoustic apparatus.
[FIG. 4] FIG. 4A is a cross-sectional view illustrating an example of a structure of a speaker, and FIG. 4B is a view illustrating a state in which the speaker is mounted to a resonance board.
[FIG. 5] FIG. 5 is a diagram showing an example of the resonance board to which a plurality of speakers are mounted.
[FIG. 6] FIG. 6 is a cross-sectional view illustrating an example of a structure of a base member.
[FIG. 7] FIG. 7 is a diagram of the resonance board according to a first modification.
[FIG. 8] FIG. 8 is a cross-sectional view of a structure of the acoustic apparatus according to a first modification.
[FIG. 9] FIG. 9 is a cross-sectional view of a structure of the acoustic apparatus according to a second modification.
[FIG. 10] FIG. 10 is a diagram of the resonance board according to a third modification.
[FIG. 11] FIG. 11 is a cross-sectional view of a structure of the acoustic apparatus according to a third modification.
Description of Embodiments

The inventor of the present invention, who developed the above-described acoustic system disclosed in Patent Literature 1, has conducted extensive studies for enhancing the physical and mental relaxation effect and health promotion effect to be obtained from the acoustic system. Through the extensive studies, the inventor of the present invention has found importance of, among the acoustic waves, acoustic waves to be transmitted to the members other than the three-dimensional network structural body. Then, in order that the acoustic waves can be effectively propagated to a human body via the filaments of the three-dimensional network structural body, the inventor of the present invention has put various ideas into members that fix the speakers, a method of fixing with these members, a method of controlling even acoustic waves to be transmitted to a floor surface, and so on. As a result, the inventor of the present invention has arrived at the present invention.

Below, an acoustic system according to an embodiment of the present invention is described with reference to the drawings.

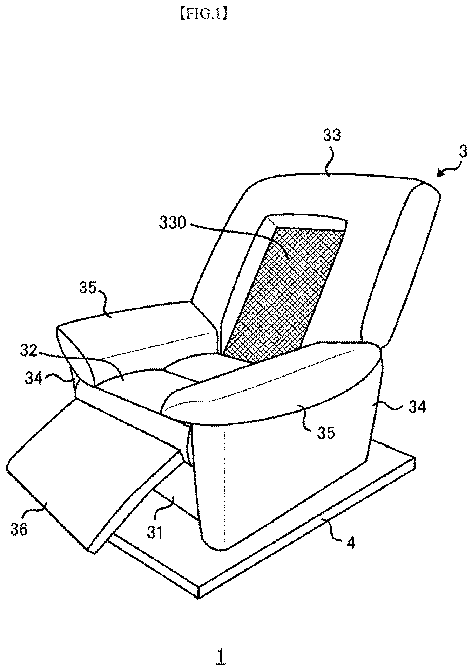

FIG. 1 is a view illustrating an example of a configuration of the acoustic system according to the embodiment. The acoustic system according to the embodiment is a system having a support surface that supports the back side of the upper half of the body of a human (especially, his/her back), and configured to transmit acoustic waves to the human body through the support surface. This acoustic system is applicable, for example, to a seat or a bed. In the example illustrated in FIG. 1, an acoustic system 1 according to this embodiment is applied to the seat.

As illustrated in FIG. 1, this acoustic system 1 includes a body portion 3 having a support surface 330 that supports at least the back of the human body, and a board-like base member 4 on which the body portion 3 is placed. The body portion 3 is a sofa seat, and includes a seat portion 31 that supports the hips, a seat-portion cushion 32 provided on an upper surface of the seat portion 31, a backrest portion 33 that supports the back, two side portions 34 that sandwich and support the seat portion 31 from its right and left side surfaces, armrest portions 35 provided respectively on upper surfaces of the two side portions 34, and a leg rest portion 36 that protrudes obliquely downward on a front side of the seat portion 31.

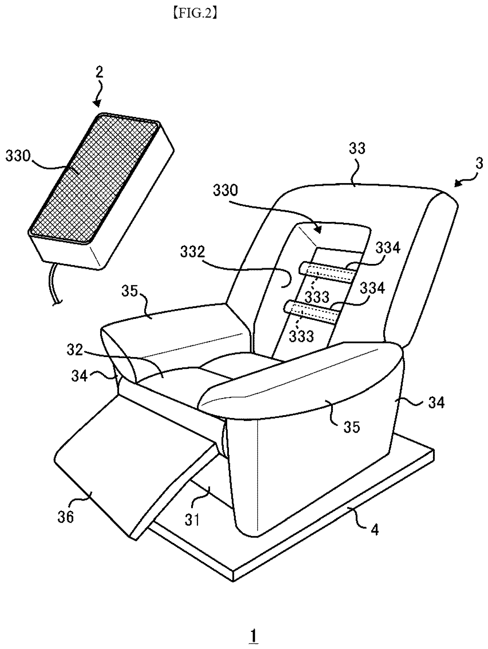

As illustrated in FIG. 2, an acoustic apparatus 2 that transmits the acoustic waves to the human body through the support surface 330 is incorporated in the backrest portion 33 of the body portion 3. FIG. 2 is a view illustrating a state in which the acoustic apparatus 2 is removed from the backrest portion 33 of the body portion 3. The backrest portion 33 includes a holding portion 331 for holding the acoustic apparatus 2. In the example illustrated in FIG. 2, the holding portion 331 includes a hole 332 formed through the backrest portion 33 from its front side to its rear side, and support frames 333 provided on the rear side in an opening portion of the hole 332. As illustrated in FIG. 1, the acoustic apparatus 2 is fitted into the hole 332. The support frames 333 support, from the rear side, an outer surface of the acoustic apparatus 2 under the fitted state in the hole 332. In the example illustrated in FIG. 2, the plurality of support frames 333 each have a bar shape and horizontally traverse the rear side in the opening portion of the hole 332.

As illustrated in FIG. 2, buffer members 334 are provided respectively at parts of the support frames 333, the parts abutting against the acoustic apparatus 2. The buffer members 334 are made of a resilient material such as glass wool. In addition, the hole 332 of the backrest portion 33 is a resilient cushion that abuts against the acoustic apparatus 2. This hole 332 resiliently sandwiches and holds side surfaces of the acoustic apparatus 2. In other words, the holding portion 331 of the backrest portion 33 includes resilient members (the buffer members 334 and the cushion of the hole 332) that hold the acoustic apparatus 2.

Since the holding portion 331 of the body portion 3 include the resilient members that hold the acoustic apparatus 2, acoustic waves generated by speakers 5A to 5G described below of the acoustic apparatus 2 are hindered from directly transmitted to the body portion 3. With this, the acoustic waves generated by these speakers are easily transmitted to the human body via a three-dimensional network structural body described below.

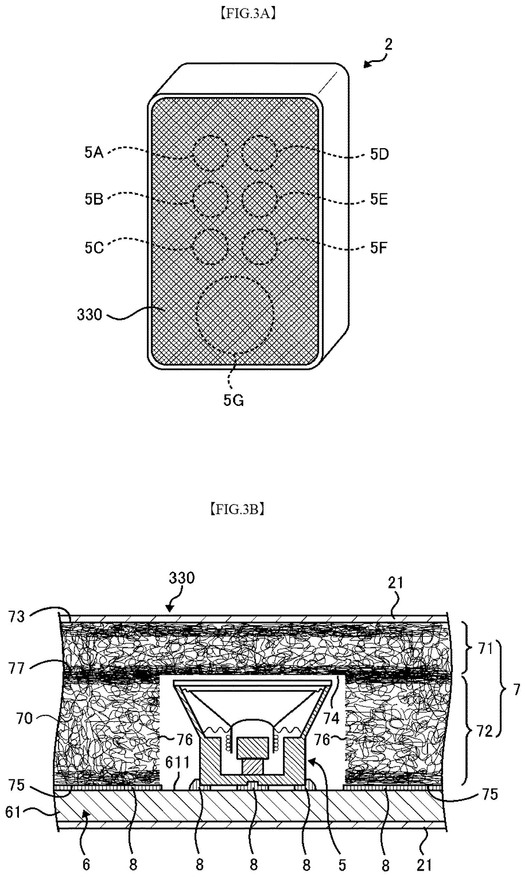

FIG. 3A is a view illustrating an example of an external appearance of the acoustic apparatus 2 according to this embodiment. In the example illustrated in FIG. 3A, the acoustic apparatus 2 has a substantially cuboid shape. When the acoustic apparatus 2 is incorporated in the backrest portion 33, another outer surface of the acoustic apparatus 2 serves as the support surface 330, and supports the back of the human body. The acoustic apparatus 2 includes a housing cover 21 that houses components therein and covers an outside. This housing cover 21 is formed, for example, of cloth, and includes a part that is formed of a mesh textile and that corresponds to the support surface 330.

In the example illustrated in FIG. 3A, the acoustic apparatus 2 includes the seven speakers 5A to 5G. The seven speakers 5A to 5G are arranged to output the acoustic waves to the outside through the support surface 330. Of the seven speakers 5A to 5G, six speakers 5A to 5F (below, sometimes collectively referred to as "speakers 5") are speakers of the same type, and only the speaker 5G is a bass speaker that is larger in size than other ones of the speakers 5. The six speakers are arrayed in two rows in a right-and-left direction as viewed in a direction perpendicular to the support surface 330, and three of the speakers 5 are arrayed in an upper-and-lower direction in each of the rows. The bass speaker 5G is disposed in the right-and-left direction substantially midway between the two rows of the speakers 5, and disposed in the upper-and-lower direction below the other ones of the speakers 5. When a user is seated in a normal posture on the body portion 3, the two rows of the speakers 5 come to both sides of the spine, and the bass speaker 5G comes near the pelvis side of the spine.

FIG. 3B is a cross-sectional view illustrating an example of a structure of the acoustic apparatus 2, in which the speaker 5 is cut out in a plane perpendicular to the support surface 330. As illustrated in FIG. 3B, the acoustic apparatus 2 includes a three-dimensional network structural body 7 that is an aggregate of filaments 70, and a board-like resonance board 6.

In the three-dimensional network structural body 7, the filaments 70 forming the aggregate are made of a resilient resin, randomly looped or curled, fused to each other, and intertwined with each other. Examples of the resin as the raw material of the filaments 70 include a thermoplastic resin such as general-purpose plastic (a polyolefin resin, a polystyrene resin, a methacrylic resin, polyvinyl chloride, or the like), or engineering plastic (polyamide, polycarbonate, saturated polyester, polyacetal, or the like). The thermoplastic resin is preferably a thermoplastic elastomer. The thermoplastic elastomer is particularly preferably, for example, the polyolefin resin such as polyethylene (PE) or polypropylene (PP), a vinyl acetate resin (VAC), ethylene-vinyl acetate copolymer (EVA), styrene butadiene styrene (SBS), or mixtures thereof. The thermoplastic resin is not limited to newly purified resins, and may be recycled resins.

When the filaments 70 are made by mixing the polyolefin resin with a vinyl-acetate-based resin of the VAC or the EVA, a mixing ratio thereof is "Polyolefin Resin : Vinyl-Acetate-Based Resin = 70 weight% to 97 weight% : 3 weight% to 30 weight%, " or preferably "80 weight% to 90 weight% : 10 weight% to 20 weight%." This is because rebound resilience decreases when the VAC or the EVA is 3 weight% or less, and thermal properties are degraded when the VAC or the EVA is 30 weight% or more.

When the filaments 70 are made by mixing the polyolefin resin and the SBS with each other, a mixing ratio thereof is "Polyolefin Resin : SBS = 50 weight% to 97 weight% : 3 weight% to 50 weight%," or preferably "70 weight% to 90 weight% : 10 weight% to 30 weight%."

The filaments 70 forming the three-dimensional network structural body 7 may be hollow, may be solid, or may be both. From a viewpoint of, for example, weight reduction, the filaments 70 are preferably hollow. When the filaments 70 have a hollow structure, a thickness of resin parts can be reduced to be smaller than that in a case where the solid filaments 70 have a uniform diameter. Thus, vibration at high frequency is easily transmitted in particular. In other words, the hollow filaments 70 have higher rigidity and less damp vibration.

When the solid filaments 70 and the hollow filaments 70 are used together, a mixing ratio thereof is preferably "Solid : Hollow = 0 to 50 : 50 to 100."

When the filaments 70 are solid, a diameter of the filaments 70 may be set to 0.3 mm to 3.0 mm, or preferably 0.7 mm to 1.0 mm. When the diameter of the solid filaments 70 is 0.3 mm or less, tenacity of the filaments 70 is low to cause an increase in fused area and a decrease in void rate. When the diameter of the solid filaments 70 is 3.0 mm or more, the tenacity of the filaments 70 is high to hinder formation of loops and to cause a decrease in fused area. As a result, strength decreases.

Meanwhile, when the filaments 70 are hollow, the diameter of the filaments 70 may be set to 1.0 mm to 3.0 mm, preferably 1.5 mm to 2.0 mm, or particularly preferably 0.9 mm to 1.3 mm. The hollow filaments 70 preferably have a hollow rate of 10% to 80%. This is because contribution to weight reduction is not made when the hollow rate is 10% or less, and cushioning properties decrease when the hollow rate is 80% or more. Note that, hollow portions to be formed in the filaments 70 may be discontinuous with each other.

From a viewpoint of securing resilience and strength that the cushion needs to have while achieving the weight reduction, the three-dimensional network structural body 7 preferably has a predetermined bulk density and a predetermined void rate. The void rate herein is expressed as follows.

$$[\text{Void Rate (\%)}] = (1 - [\text{Bulk Density}]/[\text{Resin Density}]) \times 100$$

Specifically, a bulk density of an entirety of the three-dimensional network structural body 7 may be set to 0.001 g/cm$^3$ to 0.20 g/cm$^3$, preferably 0.08 g/cm$^3$ to 0.20 g/cm$^3$, or more preferably 0.10 g/cm$^3$ to 0.18 g/cm$^3$. A void rate of the same may be set to 78% to 91%, or more preferably 80% to 88%.

The three-dimensional network structural body 7 includes high-bulk-density parts and low-bulk-density parts. In FIG. 3B, areas in which voids in a tangle are large are illustrated as the low-bulk-density parts, and areas in which the voids in the tangle are small are illustrated as the high-bulk-density parts. Of outer layers of the three-dimensional network structural body 7, at least an outer layer near the support surface 330 of the housing cover 21 and an outer layer near the resonance board 6 correspond to the high-bulk-density parts. An inner layer of the three-dimensional network structural body 7, which is sandwiched between the outer layers from above and below, corresponds to the low-bulk-density parts.

When a bulk density of the outer layers is set high, force of fusing the filaments 70 to each other is increased. Thus, pressure by application of external force is easily distributed. With this, application of high force to the speakers 5A to 5G arranged in the three-dimensional network structural body 7 can be prevented. In addition, when the bulk density of the outer layers is set high near a rear surface 75, the rear surface 75 can be reduced in unevenness and smoothed. With this, the three-dimensional network structural body 7 and the resonance board 6 can be stably mounted to each other through intermediation of a mounting material 8 described below.

Meanwhile, when a bulk density of the inner layer is set low, the cushioning properties of the three-dimensional network structural body 7 can be increased.

The bulk density of the outer layers corresponding to the high-bulk-density parts may be set to 0.2 g/cm$^3$ to

0.5 g/cm$^3$, or preferably 0.3 g/cm$^3$ to 0.4 g/cm$^3$, and a void rate of the same may be set to 44% to 77%, or preferably 56% to 67%. The bulk density of the inner layer corresponding to the low-bulk-density parts may be set to 0.01 g/cm$^3$ to 0.15 g/cm$^3$, or preferably 0.03 g/cm$^3$ to 0.05 g/cm$^3$, and a void rate of the same may be set to 83% to 99%, or preferably 94% to 97%.

A thickness of the three-dimensional network structural body 7 is not limited in particular as long as the strength and the resilience that are necessary for supporting the human body can be exerted. This thickness is set to a thickness that is necessary at least for installing the speakers 5A to 5G in the three-dimensional network structural body 7. As an example, the thickness of the three-dimensional network structural body 7 may be set to 60 mm to 100 mm, or preferably 70 mm to 80 mm. The three-dimensional network structural body 7 may be formed into arbitrary shapes or sizes by methods such as fusion cutting, mechanical cutting, and hot pressing.

As illustrated in FIG. 3B, the three-dimensional network structural body 7 has a front surface 73 that is held in indirect contact with the human body through intermediation of the support surface 330 of the housing cover 21, and an inner surface 74 that faces the front surface 73. The inner surface 74 faces a vibrating portion 51 described below of the speaker 5.

Further, as illustrated in FIG. 3B, the three-dimensional network structural body 7 has the rear surface 75 that faces the front surface 73. The rear surface 75 is farther from the front surface 73 than from the inner surface 74, and is mounted to one side surface 611 of the resonance board 6 through intermediation of the mounting material 8.

Still further, as illustrated in FIG. 3B, the three-dimensional network structural body 7 has inner side surfaces 76 that surround the speaker 5 between the inner surface 74 and the rear surface 75. The speaker 5 is housed in an interior space surrounded by the inner surface 74 and the inner side surfaces 76 of the three-dimensional network structural body 7 and by the surface 611 of the resonance board 6.

The three-dimensional network structural body 7 exemplified in FIG. 3B is formed by superimposing a front-surface-side member 71 near the support surface 330 of the housing cover 21 and an inside member 72 near the resonance board 6 on each other. The front-surface-side member 71 and the inside member 72 each have a cuboid shape, and the inside member 72 is larger in thickness than the front-surface-side member 71.

The inside member 72 has through-holes for arranging therein the speakers 5A to 5G. The through-holes of the inside member 72, in each of which the speaker 5 is disposed, form the inner side surfaces 76 that surround a corresponding one of the speakers 5. The front-surface-side member 71 closes an opening portion of each of the through-holes on a side where the support surface 330 is present. A surface of the front-surface-side member 71 on a side near the support surface 330 is the above-described front surface 73, and another surface of the front-surface-side member 71 on a side opposite to the front surface 73 (surface that faces the front surface 73) is the above-described inner surface 74. At a position on the through-hole of the inside member 72, the inner surface 74 of the front-surface-side member 71 faces the vibrating portion 51 of the speaker 5 arranged in this through-hole. At positions out of the through-hole, the inner surface 74 of the front-surface-side member 71 abuts against an inner surface 77 of the inside member 72. The inner surface 74 and the inner surface 77 may be mounted through intermediation of the mounting material 8. In the example illustrated in FIG. 3B, an outer layer of the front-surface-side member 71 near the inner surface 74 and an outer layer of the inside member 72 near the inner surface 77 each correspond to the high-bulk-density part. A surface of the inside member 72 on a side opposite to the inner surface 77 (surface that faces the inner surface 77) is the above-described rear surface 75.

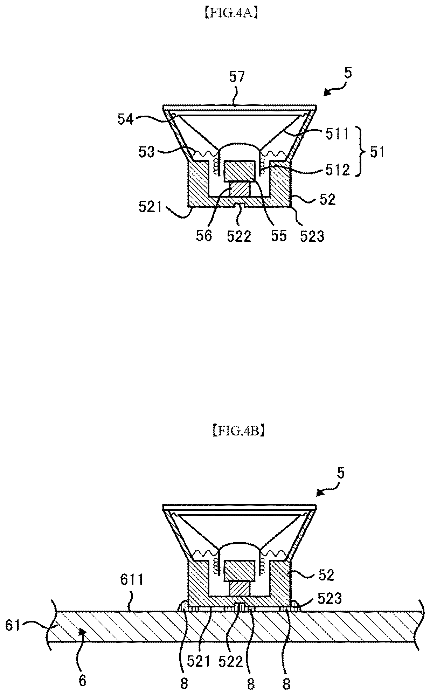

FIG. 4A is a cross-sectional view illustrating an example of a structure of the speaker 5. The speaker 5 includes the vibrating portion 51 that faces the inner surface 74 of the three-dimensional network structural body 7, and a frame portion 52 that holds the vibrating portion 51 while allowing the vibrating portion 51 to vibrate. In the example illustrated in FIG. 4A, the vibrating portion 51 includes a cone diaphragm 511 and a voice coil 512 mounted near a central portion of the diaphragm 511. On a side near the central portion, the diaphragm 511 is connected to the frame portion 52 through intermediation of a ring-like support portion 53. On another side near an outer rim, the diaphragm 511 is connected to the frame portion 52 through intermediation of another ring-like support portion 54 that is larger in diameter than the support portion 53. Both the support portion 53 and the support portion 54 are resiliently deformable, and allow the diaphragm 511 to vibrate relative to the frame portion 52. A columnar yoke 55 is arranged in the voice coil 512. The frame portion 52 is formed of the same magnetic body as that of the yoke 55 such that magnetic flux is generated between the yoke 55 and the frame portion 52 by a magnet 56. This magnetic flux acts on current in the voice coil 512 disposed between the frame portion 52 and the yoke 55. With this, electromagnetic force in accordance with an audio signal to be superimposed on the current acts on the voice coil 512 to cause the diaphragm 511 to vibrate in response to the audio signal. The diaphragm 511 vibrates in a direction substantially perpendicular to the front surface 73 and the inner surface 74 of the three-dimensional network structural body 7, which are planes substantially parallel to each other.

In the example illustrated in FIG. 4A, the frame portion 52 has a substantially-circular flat bottom surface 521. A recessed portion 522 is formed at a center of the bottom surface 521.

FIG. 4B is a view illustrating a state in which the speaker 5 is mounted to the resonance board 6. As illustrated in FIG. 4B, the frame portion 52 of the speaker 5 is mounted to the one side surface 611 of the resonance board 6 through intermediation of the mounting material 8 that is resilient. The mounting material 8 preferably has appropriate resilience also under a cured state. For example, the thermoplastic elastomer including the ethylene-vinyl acetate copolymer (EVA) and glue are preferable.

When the frame portion 52 and the resonance board 6 are mounted to each other through intermediation of the resilient mounting material 8, acoustic waves (specifically, components at high frequency) can be further prevented from being propagated from the frame portion 52 to the resonance board 6 than in a case where these members are fixed to each other by fastening with bolts or the like. Thus, the acoustic waves to be generated by the speaker 5 (components at relatively high frequency) can be effectively propagated to the human body via the filaments 70 of the three-dimensional network structural body 7.

In addition, components at relatively low frequency of the acoustic waves that are propagated from the frame portion 52 to the resonance board 6 via the mounting material 8 easily resonate when the resonance board 6 has a size, specifically, a horizontal width and a height (approximately from several cm to several tens of cm) that facilitate arrangement in face of the back of the human body. Thus, the components are easily amplified by the resonance board 6 and then transmitted to the three-dimensional network structural body 7. In this way, the components at the relatively low frequency of the acoustic waves to be generated by the speaker 5 can also be effectively propagated to the human body via the filaments 70 of the three-dimensional network structural body 7. In particular, when the rear surface 75 of the three-dimensional network structural body 7 is mounted to the surface 611 of the resonance board 6 through intermediation of the mounting material 8, the acoustic waves amplified by the resonance board 6 are easily transmitted to the three-dimensional network structural body 7. In this way, the acoustic waves can be further effectively transmitted to the human body via the three-dimensional network structural body 7.

In the example illustrated in FIG. 4B, the resonance board 6 is formed of a wooden board 61. Examples of a material of the wooden board 61 include plywood such as lumber core. The frame portion 52 of each of the speakers 5 is mounted to the one side surface 611 of the wooden board 61 through intermediation of the mounting material 8.

As illustrated, for example, in FIG. 4B, the mounting material 8 includes one part applied in the recessed portion 522 of the bottom surface 521, and another part applied at least to a part of an outer rim portion 523 of the bottom surface 521, and the frame portion 52 is mounted to the one side surface 611 of the resonance board 6 through intermediation of these parts of the mounting material 8. When the mounting material 8 is applied into the recessed portion 522 of the bottom surface 521, a central part of the bottom surface 521 of the frame portion 52 and the surface 611 of the wooden board 61 can be reliably prevented from being held in direct contact with each other. In addition, along the outer rim portion 523 of the bottom surface 521, a range in which the frame portion 52 is mounted to the surface 611 of the wooden board 61 can be easily adjusted. Thus, for example, intensity and the frequency of the acoustic waves to be transmitted to the resonance board 6 via the frame portion 52 can be easily adjusted.

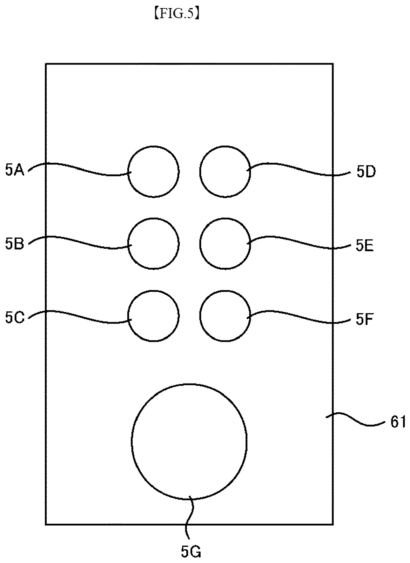

FIG. 5 is a diagram showing an example of the resonance board 6 (wooden board 61) to which the plurality of speakers (5A to 5G) are mounted as viewed in a direction perpendicular to a plane of the resonance board 6 (wooden board 61). The wooden board 61 shown in FIG. 5 has a rectangular planar shape. As an example, the wooden board 61 has a width of 400 mm, a length of 650 mm, and a thickness of 13 mm. The plurality of speakers (5A to 5G) are mounted to the single wooden board 61.

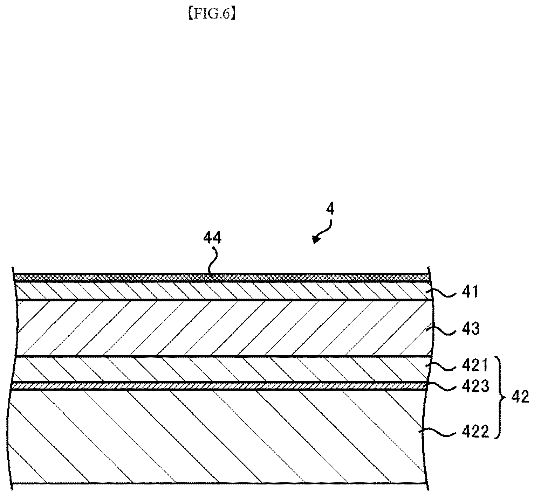

FIG. 6 is a cross-sectional view illustrating an example of a structure of the base member 4 on which the body portion 3 is placed, in which the base member 4 is cut out in a plane along a vertical direction. As illustrated, for example, in FIG. 6, the base member 4 includes a first board member 41 and a second board member 42, each of which is a board-like member, and a buffer member 43. The second board member 42 is arranged under the first board member 41, and the buffer member 43 is inserted between the first board member 41 and the second board member 42. The first board member 41 is a wooden board member such as the plywood. As an example, the first board member 41 has a thickness of 55 mm. The buffer member 43 is made of the resilient material such as the glass wool or rock wool. As an example, the buffer member 43 has a thickness of 20 mm. As an example, the first board member 41 and the second board member 42 each have a planar rectangular shape of the same size, specifically, have a longitudinal length of 800 mm and a transverse length of 840 mm. An upper surface of the first board member 41 is covered with a cover member 44 such as a carpet.

The acoustic waves generated by the speakers 5A to 5G of the acoustic apparatus 2 are partially propagated to the base member 4 via the body portion 3 or the human body. However, since the first board member 41 of the base member 4 is supported from below by the second board member 42 through intermediation of the buffer member 43, an entirety of the first board number 41 easily vibrates relative to the second board number 42 and the floor surface. Thus, the acoustic waves are prevented from being propagated to the floor surface via the first board member 41. In addition, when the acoustic waves (specifically, components at low frequency) vibrate the first board member 41, this vibration is easily transmitted to the human body via toes that touch the base member 4 or the body portion 3. Thus, comfortable vibration to be caused by the acoustic waves generated by the acoustic apparatus 2 can be delivered to an entirety of the body of the user.

As illustrated, for example, in FIG. 6, the second board member 42 includes a wooden upper-board member 421 that faces the buffer member 43, a wooden lower-board member 422 that is arranged under the upper board member 421, and an elastomeric sheet 423 that is inserted between the upper board member 421 and the lower board member 422. As an example, the upper board member 421 is a medium density fiberboard (MDF) having a thickness of 12 mm, the lower board member 422 is a particle board having the thickness of 20 mm, and the elastomeric sheet 423 is a rubber sheet having a thickness of 3 mm. Such a structure enables the elastomeric sheet 423 such as rubber to reduce the propagation of the vibration from the upper board member 421 to the lower board member 422. Thus, the vibration of the first board member 41 to be caused by the acoustic waves can be prevented from being propagated to the floor surface.

When the user uses the above-described acoustic system 1, the user sits on the seat portion 31 of the body portion 3 such that his/her back abuts against the support surface 330. In this state, the acoustic waves such as those of music or environmental sound are generated from the speakers 5A to 5G. At this time, when the frequency of the acoustic waves to be generated from speakers 5 that come to a side lower than his/her back among the speakers 5 is set lower, physiological pleasure and massage effect can be obtained.

According to this embodiment, the frame portion 52 of the speaker 5 and the resonance board 6 are mounted to each other through intermediation of the resilient mounting material 8. With this, the acoustic waves at high frequency can be prevented from being propagated the resonance board 6 via the frame portion 52, and the acoustic waves at low frequency, which have been propagated to the resonance board 6 via the frame portion 52, can easily resonate in the resonance board 6. Thus, the acoustic waves can be effectively propagated to the human body via the filaments 70 of the three-dimensional network structural body 7.

Further, according to this embodiment, the first board member 41 of the base member 4 is supported from below by the second board member 42 through intermediation of the buffer member 43. With this, the entirety of the first board member 41 easily vibrates relative to the second board member 42 and the floor surface. Thus, the vibration to be caused by the acoustic waves generated by the acoustic apparatus 2 can be transmitted to the entirety of the body.

Yet further, according to this embodiment, the holding portion 331 of the body portion 3 includes the resilient members that hold the acoustic apparatus 2. With this, the acoustic waves generated by the speakers 5 of the acoustic apparatus 2 are prevented from being propagated toward the body portion 3. Thus, the acoustic waves via the filaments 70 of the three-dimensional network structural body 7 are easily propagated to the human body.

Next, a first modification of the acoustic apparatus 2 according to this embodiment is described.

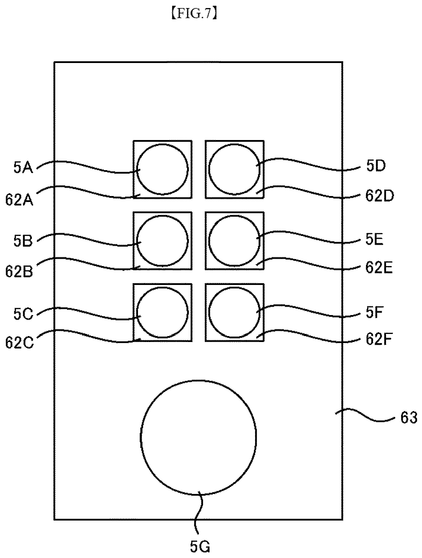

FIG. 7 is a diagram of the resonance board 6 according to the first modification as viewed in the direction perpendicular to the plane of the resonance board 6. The resonance board 6 shown in FIG. 7 includes six wooden boards 62A to 62F (below, sometimes collectively referred to as "wooden boards 62") that correspond one on one to six speakers 5A to 5F, and a board member 63 to which these wooden boards 62A to 62F are mounted. In an example shown in FIG. 7, the wooden boards 62A to 62F have rectangular planar shapes that are substantially congruent with each other. In addition, in the example shown in FIG. 7, the board member 63 also has a rectangular planar shape that is larger in size than those of the wooden boards 62A to 62F. As an example, the wooden boards 62A to 62F each have a width of 80 mm, a length of 80 mm, and the thickness of 13 mm, and the board member 63 has the width of 400 mm, the length of 650 mm, and a thickness of 25 mm. The wooden boards 62A to 62F and the board member 63 are each formed of the plywood such as lumber core. The bass speaker 5G is mounted, for example, directly to the board member 63 without intermediation of the wooden board 62.

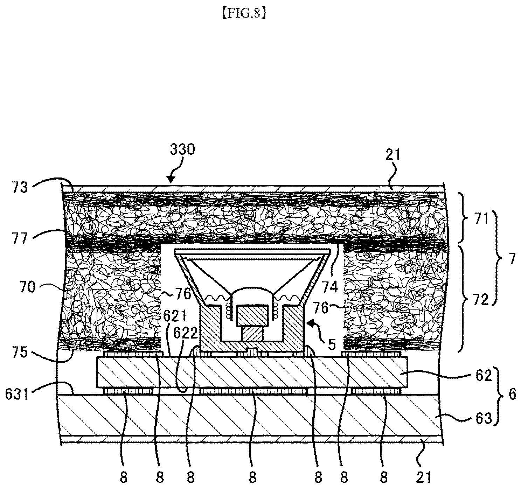

FIG. 8 is a cross-sectional view of a structure of the acoustic apparatus 2 according to the first modification. As illustrated in FIG. 8, the frame portion 52 of each of the speakers 5 is mounted to one side surface 621 of a corresponding one of the wooden boards 62 through intermediation of the mounting material 8. The wooden boards 62 mounted respectively to the frame portions 52 each have another side surface 622 that faces the one side surface 621 of the corresponding one of the wooden boards 62, and that is mounted to one side surface 631 of the board member 63 through intermediation of the mounting material 8.

According to the first modification, the plurality of speakers 5 are mounted respectively to the wooden boards 62 independent of each other. Thus, the wooden boards 62 to be provided respectively to the speakers 5 can have appropriate sizes in which the resonance is easily generated. In addition, the acoustic waves generated by each of the speakers 5 easily resonate in a corresponding one of the independent wooden boards 62. With this, the acoustic waves generated by different ones of the speakers 5 can be prevented from being cancelled out each other unlike in the single wooden board 62.

Next, a second modification of the acoustic apparatus 2 according to this embodiment is described.

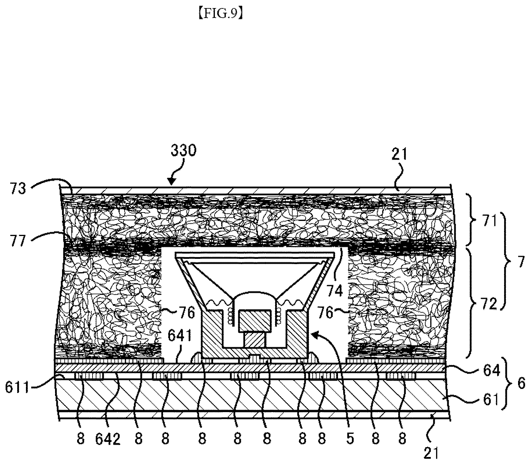

FIG. 9 is a cross-sectional view of a structure of the acoustic apparatus 2 according to the second modification. The resonance board 6 of the acoustic apparatus 2 illustrated in FIG. 9 includes a metal board 64 in addition to the wooden board 61 that is similar to that of the resonance board 6 of the acoustic apparatus 2 illustrated in FIG. 3B. The metal board 64 covers at least a part of the one side surface 611 of the wooden board 61, and is disposed on a side where the support surface 330 is present relative to the wooden board 61. As materials of the metal board 64, metals such as copper and iron, or alloys made of a plurality of metals may be used. As an example, the metal board 64 is a rectangular copper board having a width of 200 mm, a length of 300 mm, and a thickness of 0.3 mm.

The frame portion 52 of the speaker 5 is mounted to one side surface 641 of the metal board 64 through intermediation of the mounting material 8. In addition, the rear surface 75 of the three-dimensional network structural body 7 is also mounted to the one side surface 641 of the metal board 64 through intermediation of the mounting material 8. A method of mounting the frame portion 52 to the metal board 64 is similar to the method of mounting the frame portion 52 to the wooden board 61 (FIG. 4B). In other words, the mounting material 8 includes the one part applied in the recessed portion 522 of the bottom surface 521, and the other part applied at least to the part of the outer rim portion 523 of the bottom surface 521, and the frame portion 52 is mounted to the one side surface 641 of the metal board 64 through intermediation of these parts of the mounting material 8.

The metal board 64 mounted to the frame portion 52 has another side surface 642 that faces the one side surface 641 of the metal board 64, and that is mounted to the one side surface 611 of the wooden board 61 through intermediation of the mounting material 8. As illustrated in FIG. 9, the mounting material 8 includes parts applied to a plurality of points away from each other on the other side surface 642 of the metal board 64, and this surface 642 is mounted to the one side surface 611 of the wooden board 61 through intermediation of these parts of the mounting material 8.

In such a structure of the acoustic apparatus 2 according to the second modification, acoustic waves propagated from the frame portion 52 to the metal board 64 via the mounting material 8 resonate in the metal board 64. In addition, these acoustic waves are propagated from the metal board 64 to the wooden board 61 via the mounting material 8, and then resonate in the wooden board 61. In this way, the acoustic waves resonate at respective different resonance frequencies of the metal board 64 and the wooden board 61, and then are transmitted to the human body via the three-dimensional network structural body 7. Thus, the acoustic waves via the filaments 70 of the three-dimensional network structural body 7 can be further effectively transmitted to the human body. In addition, the acoustic waves that have resonated at the different resonance frequencies are easily transmitted to the human body, and hence relaxation effects and health promoting effects at the different frequencies of the acoustic waves can be synergistically given to the human body.

Next, a third modification of the acoustic apparatus 2 according to this embodiment is described.

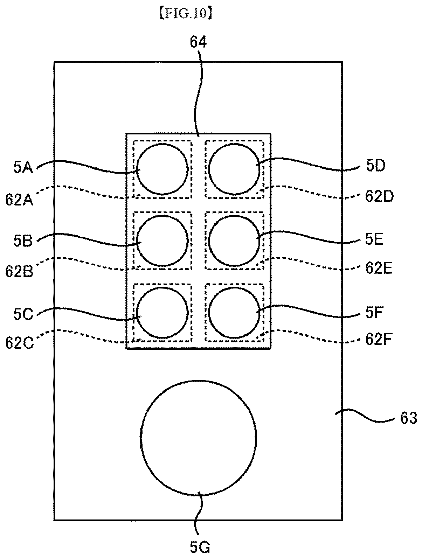

FIG. 10 is a diagram of the resonance board 6 according to the third modification as viewed in the direction perpendicular to the plane of the resonance board 6. The resonance board 6 shown in FIG. 10 includes the metal board 64 in addition to the wooden boards 62A to 62F (wooden boards 62) and the board member 63 that are similar to those of the resonance board 6 according to the first modification shown in FIG. 7. As materials of the metal board 64, similar to those of the metal board 64 according to the second modification, the metals such as copper and iron, or the alloys made of a plurality of metals may be used. In the example shown in FIG. 10, the metal board 64 has a rectangular planar shape, and is interposed between the six wooden boards 62A to 62F (wooden boards 62) and the six speakers 5A to 5F (speakers 5).

As an example, the metal board 64 has the width of 200 mm, the length of 300 mm, and the thickness of 0.3 mm. In addition, as an example, the wooden boards 62A to 62F each have the width of 80 mm, the length of 80 mm, and the thickness of 13 mm, and the board member 63 has the width of 400 mm, the length of 650 mm, and the thickness of 25 mm. The wooden boards 62A to 62F and the board member 63 are each formed of the plywood such as lumber core. The bass speaker 5G is mounted, for example, directly to the board member 63 without intermediation of the wooden board 62.

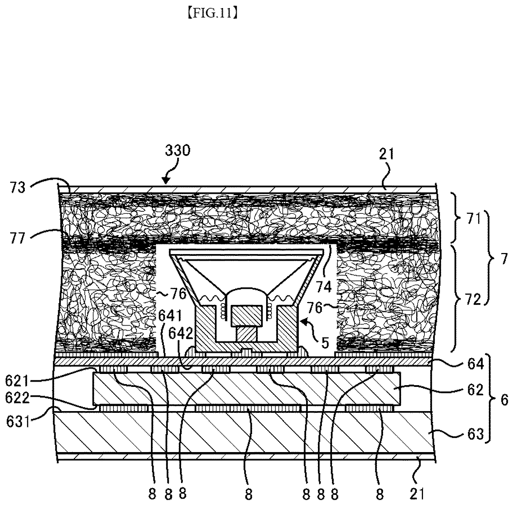

FIG. 11 is a cross-sectional view of a structure of the acoustic apparatus 2 according to a third modification.

The frame portion 52 of the speaker 5 is mounted to the one side surface 641 of the metal board 64 through intermediation of the mounting material 8. In addition, the rear surface 75 of the three-dimensional network structural body 7 is also mounted to the one side surface 641 of the metal board 64 through intermediation of the mounting material 8. The method of mounting the frame portion 52 to the metal board 64 is similar to the method of mounting the frame portion 52 to the wooden board 61 (FIG. 4B). In other words, the mounting material 8 includes the one part applied in the recessed portion 522 of the bottom surface 521, and the other part applied at least to the part of the outer rim portion 523 of the bottom surface 521, and the frame portion 52 is mounted to the one side surface 641 of the metal board 64 through intermediation of these parts of the mounting material 8.

The metal board 64 mounted to the frame portion 52 has the other side surface 642 that faces the one side surface 641 of the metal board 64, and that is mounted to the one side surface 621 of the wooden board 62 through intermediation of the mounting material 8. As illustrated in FIG. 11, the mounting material 8 include the parts applied to a plurality of points away from each other on the other side surface 642 of the metal board 64, and this surface 642 is mounted to the one side surface 621 of the wooden board 62 through intermediation of these parts of the mounting material 8.

As illustrated in FIG. 11, one wooden board 62 of the wooden boards 62 and the frame portion 52 of one speaker 5 of the speakers 5, the one speaker 5 corresponding to the one wooden board 62, are mounted to both the side surfaces of the metal board 64 in a manner that the one speaker 5 and the one wooden board 62 face each other across the metal board 64. As illustrated in FIG. 10, the one wooden board 62 and the one speaker 5 corresponding to each other are disposed at a position where the one wooden board 62 and the one speaker 5 are superimposed on each other as viewed in a direction perpendicular to a plane of the metal board 64. The wooden board 62 mounted to the metal board 64 has the other side surface 622 that faces the one side surface 621 of the wooden board 62, and that is mounted to the one side surface 631 of the board member 63 through intermediation of the mounting material 8.

In such a structure of the acoustic apparatus 2 according to the third modification, the acoustic waves generated by the plurality of speakers 5 are easily propagated respectively to the independent wooden boards 62 disposed across the metal board 64. Thus, the wooden boards 62 to be provided respectively to the speakers 5 can have appropriate sizes in which the resonance is easily generated. In addition, among the acoustic waves generated by each of the speakers 5, acoustic waves that are propagated from the one side surface of the metal board 64 to the other side surface of the same easily resonate in a corresponding one of the independent wooden boards 62. With this, the acoustic waves generated by different ones of the speakers 5 can be prevented from being cancelled out each other unlike in the single wooden board 62.

Note that, the present invention is not limited only to the above-described embodiment, and may include other variations.

For example, although the seven speakers (5A to 5G) are provided in the above-described embodiment, six or less speakers or eight or more speakers may be provided. In addition, the speakers in the acoustic apparatus are not limited to the cone type as illustrated in FIG. 4B, and speakers of other types may be used.

The sizes, properties of the materials, types of the materials, and the like of the members described in the above-described embodiment are merely examples, and may be changed as appropriate.

Reference Signs List

| | |
|---|---|
| 1 | acoustic system |
| 2 | acoustic apparatus |
| 21 | housing cover |
| 3 | body portion |
| 31 | seat portion |
| 32 | seat-portion cushion |
| 33 | backrest portion |
| 330 | support surface |
| 331 | holding portion |
| 332 | hole |
| 333 | support frame |
| 334 | buffer member |
| 34 | side portion |
| 35 | armrest portion |
| 36 | leg rest portion |
| 4 | base member |
| 41 | first board member |
| 42 | second board member |
| 421 | upper board member |
| 422 | lower board member |
| 423 | elastomeric sheet |
| 43 | buffer member |
| 44 | cover member |
| 5 | speaker |
| 5A to 5G | speaker |
| 51 | vibrating portion |
| 511 | diaphragm |
| 512 | voice coil |
| 52 | frame portion |
| 521 | bottom surface |
| 522 | recessed portion |
| 523 | outer rim portion |
| 53, 54 | support portion |

| 55 | yoke |
| 56 | magnet |
| 57 | protection cover |
| 6 | resonance board |
| 61 | wooden board |
| 611 | surface |
| 62A to 62F, 62 | wooden board |
| 621, 622 | surface |
| 63 | board member |
| 631 | surface |
| 64 | metal board |
| 641, 642 | surface |
| 7 | three-dimensional network structural body |
| 70 | filament |
| 71 | front-surface-side member |
| 72 | inside member |
| 73 | front surface |
| 74, 77 | inner surface |
| 75 | rear surface |
| 76 | inner side surface |
| 8 | mounting material 8 |

**Claims**

1.  An acoustic apparatus that transmits acoustic waves to a human body, the acoustic apparatus comprising:

    a three-dimensional network structural body that is an aggregate of filaments that are

        made of a resilient resin,
        each randomly looped or curled,
        fused to each other, and
        intertwined with each other;

        a plurality of speakers that generate the acoustic waves; and
        a board-like resonance board,
        wherein the three-dimensional network structural body has

        a front surface that is held in direct or indirect contact with the human body, and
        an inner surface that faces the front surface,

        wherein the plurality of speakers respectively include

        vibrating portions that face the inner surface of the three-dimensional network structural body, and
        frame portions that respectively hold the vibrating portions while allowing the vibrating portions to vibrate,

        wherein the frame portions are mounted to one side surface of the board-like resonance board through intermediation of a resilient mounting material,
        wherein the board-like resonance board includes

        a plurality of wooden boards that correspond one on one to the plurality of speakers, and
        a board member to which the plurality of wooden boards are mounted,

        wherein the frame portions of the plurality of speakers are mounted respectively to one side surfaces of corresponding ones of the plurality of wooden boards through intermediation of the resilient mounting material, and
        wherein the plurality of wooden boards mounted respectively to the frame portions each have another side surface that faces the one side surface of a corresponding one of the plurality of wooden boards, and that is mounted to one side surface of the board member through intermediation of the resilient mounting material.

**2.** The acoustic apparatus according to Claim 1,

wherein the frame portions each have a circular-flat bottom surface,
wherein a recessed portion is formed at a center of the circular-flat bottom surface,
wherein the resilient mounting material includes

one part applied in the recessed portion, and
another part applied at least to a part of an outer rim portion of the circular-flat bottom surface, and

wherein the frame portions are mounted respectively to the one side surfaces of the corresponding ones of the plurality of wooden boards through intermediation of the one part and the other part of the resilient mounting material.

**3.** An acoustic apparatus that transmits acoustic waves to a human body, the acoustic apparatus comprising:

a three-dimensional network structural body that is an aggregate of filaments that are

made of a resilient resin,
each randomly looped or curled,
fused to each other, and
intertwined with each other;

an at least one speaker that generates the acoustic waves; and
a board-like resonance board,
wherein the three-dimensional network structural body has

a front surface that is held in direct or indirect contact with the human body, and
an inner surface that faces the front surface,

wherein the at least one speaker includes

a vibrating portion that faces the inner surface of the three-dimensional network structural body, and
a frame portion that holds the vibrating portion while allowing the vibrating portion to vibrate,

wherein the frame portion is mounted to one side surface of the board-like resonance board through intermediation of a resilient mounting material,
wherein the board-like resonance board includes

a wooden board and
a metal board,

wherein the metal board covers at least a part of one side surface of the wooden board, and
wherein the frame portion is mounted to one side surface of the metal board through intermediation of the resilient mounting material.

**4.** The acoustic apparatus according to Claim 3,

wherein the frame portion has a circular-flat bottom surface,
wherein a recessed portion is formed at a center of the circular-flat bottom surface,
wherein the resilient mounting material includes

one part applied in the recessed portion, and
another part applied at least to a part of an outer rim portion of the circular-flat bottom surface, and

wherein the frame portion is mounted to the one side surface of the metal board through intermediation of the one part and the other part of the resilient mounting material.

**5.** The acoustic apparatus according to Claim 3 or 4,

wherein the metal board mounted to the frame portion has another side surface that faces the one side surface of the metal board, and that is mounted to the one side surface of the wooden board through intermediation of the resilient mounting material.

6. The acoustic apparatus according to Claim 5,

wherein the resilient mounting material includes parts applied to a plurality of points away from each other on the other side surface of the metal board, and
wherein the other side surface of the metal board is mounted to the one side surface of the wooden board through intermediation of the parts of the resilient mounting material.

7. The acoustic apparatus according to Claim 5 or 6,

wherein the at least one speaker includes a plurality of speakers,
wherein the wooden board of the board-like resonance board includes a plurality of wooden boards that correspond one on one to the plurality of speakers,
wherein the board-like resonance board includes a board member to which the plurality of wooden boards are mounted,
wherein one wooden board of the plurality of wooden boards and the frame portion of one speaker of the plurality of speakers, the one speaker corresponding to the one wooden board, are mounted to both the one side surface and the other side surface of the metal board in a manner that the one speaker and the one wooden board face each other across the metal board, and
wherein the other side surface of the one wooden board mounted to the metal board

    faces the one side surface of the one wooden board, and
    is mounted to one side surface of the board member through intermediation of the resilient mounting material.

8. The acoustic apparatus according to any of Claims 3 to 7,
wherein the metal board is a copper board.

9. The acoustic apparatus according to any one of Claims 1 to 8,

wherein the three-dimensional network structural body has a rear surface

    that faces the front surface, and
    that is farther from the front surface than from the inner surface, and
    inner side surfaces that surround the speaker between the inner surface and the rear surface, and

wherein the speaker is housed in an interior space surrounded

    by the inner surface and the inner side surfaces of the three-dimensional network structural body, and
    by the one side surface of the board-like resonance board.

10. The acoustic apparatus according to Claim 9,
wherein the rear surface of the three-dimensional network structural body is mounted to the one side surface of the board-like resonance board through intermediation of the resilient mounting material.

11. The acoustic apparatus according to any one of Claims 1 to 10,
wherein the resilient mounting material is

    a thermoplastic elastomer including ethylene-vinyl acetate copolymer, or
    glue.

12. An acoustic system, comprising:

    a body portion having a support surface that supports at least the back of a human body; and
    a board-like base member on which the body portion is placed,
    wherein the body portion includes

the acoustic apparatus according to any of Claims 1 to 11, the acoustic apparatus transmitting the acoustic waves to the human body through the support surface, and

a holding portion that holds the acoustic apparatus, and

wherein the base member includes

a first board member,
a second board member that is arranged under the first board member, and
a resilient buffer member that is inserted between the first board member and the second board member.

13. The acoustic system according to Claim 12,
wherein the first board member is a wooden board member.

14. The acoustic system according to Claim 12 or 13,
wherein the resilient buffer member is made of

glass wool or
rock wool.

15. The acoustic system according to any one of Claims 12 to 14,
wherein the second board member includes

a wooden upper-board member that faces the resilient buffer member,
a wooden lower-board member that is arranged under the wooden upper-board member, and
an elastomeric sheet that is inserted between the wooden upper-board member and the wooden lower-board member.

16. The acoustic system according to any one of Claims 12 to 15,
wherein the holding portion includes a resilient member that holds the acoustic apparatus.

17. An acoustic apparatus that transmits acoustic waves to a human body, the acoustic apparatus comprising:

a three-dimensional network structural body that is an aggregate of filaments that are

made of a resilient resin,
each randomly looped or curled,
fused to each other, and
intertwined with each other;

an at least one speaker that generates the acoustic waves; and
a board-like resonance board,
wherein the three-dimensional network structural body has

a front surface that is held in direct or indirect contact with the human body, and
an inner surface that faces the front surface,

wherein the at least one speaker includes

a vibrating portion that faces the inner surface of the three-dimensional network structural body, and
a frame portion that holds the vibrating portion while allowing the vibrating portion to vibrate, and

wherein the frame portion is mounted to one side surface of the board-like resonance board through intermediation of a resilient mounting material.

18. The acoustic apparatus according to Claim 17,

wherein the board-like resonance board includes a wooden board, and
wherein the frame portion is mounted to one side surface of the wooden board through intermediation of the resilient mounting material.

19. The acoustic apparatus according to Claim 18,

wherein the frame portion has a circular-flat bottom surface,
wherein a recessed portion is formed at a center of the circular-flat bottom surface,
wherein the resilient mounting material includes

one part applied in the recessed portion, and
another part applied at least to a part of an outer rim portion of the circular-flat bottom surface, and

wherein the frame portion is mounted to the one side surface of the wooden board through intermediation of the one part and the other part of the resilient mounting material.

【FIG.1】

1

【FIG.2】

1

【FIG.3A】

2

5A — 5D
5B — 5E
5C — 5F

330

5G

【FIG.3B】

330        21

73
77          71
70          7
74
76    76    72
611
75          75
61
6   8   8   8   8   5   8   21

【FIG.4A】

【FIG.4B】

【FIG.5】

【FIG.6】

【FIG.7】

【FIG.8】

【FIG.9】

【FIG.10】

64

5A
62A

5B
62B

5C
62C

5D
62D

5E
62E

5F
62F

63

5G

【FIG.11】

**INTERNATIONAL SEARCH REPORT**

| International application No. |
| --- |
| PCT/JP2021/025150 |

A. CLASSIFICATION OF SUBJECT MATTER
Int. Cl. H04R1/00(2006.01)i, H04R1/02(2006.01)i, H04R1/28(2006.01)i,
G10K11/24(2006.01)i, A47C27/00(2006.01)i
FI: H04R1/00 310G, A47C27/00 E, G10K11/24, H04R1/02 105B, H04R1/28 310Z
According to International Patent Classification (IPC) or to both national classification and IPC

B. FIELDS SEARCHED

Minimum documentation searched (classification system followed by classification symbols)
Int. Cl. H04R1/00, H04R1/02, H04R1/28, G10K11/24, A47C27/00

Documentation searched other than minimum documentation to the extent that such documents are included in the fields searched
Published examined utility model applications of Japan    1922-1996
Published unexamined utility model applications of Japan    1971-2021
Registered utility model specifications of Japan    1996-2021
Published registered utility model applications of Japan    1994-2021

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | WO 2005/030011 A1 (EIN CO., LTD. TECHNICAL CENTER) 07 April 2005 (2005-04-07), fig. 4 (b) | 1-19 |
| A | WO 2006/109389 A1 (PIONEER CORP.) 19 October 2006 (2006-10-19), fig. 1-4, 1-5 | 1-19 |
| A | JP 9-247780 A (MATSUSHITA ELECTRIC INDUSTRIAL CO., LTD.) 19 September 1997 (1997-09-19), fig. 2 | 1-19 |
| A | JP 8-033077 A (TOSHIBA CORP.) 02 February 1996 (1996-02-02), fig. 1, 2 | 1-19 |

☐ Further documents are listed in the continuation of Box C.     ☒ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 01.09.2021 | 14.09.2021 |

| Name and mailing address of the ISA/<br>    Japan Patent Office<br>    3-4-3, Kasumigaseki, Chiyoda-ku,<br>    Tokyo 100-8915, Japan | Authorized officer<br><br>Telephone No. |
| --- | --- |

Form PCT/ISA/210 (second sheet) (January 2015)

**INTERNATIONAL SEARCH REPORT**
Information on patent family members

International application No.

PCT/JP2021/025150

| Patent Documents referred to in the Report | Publication Date | Patent Family | Publication Date |
|---|---|---|---|
| WO 2005/030011 A1 | 07.04.2005 | US 2007/0086612 A1 fig. 4 (b) EP 1683446 A1 CN 1859862 A KR 10-2006-0106818 A | |
| WO 2006/109389 A1 | 19.10.2006 | US 2009/0067657 A1 fig. 1-4, 1-5 | |
| JP 9-247780 A | 19.09.1997 | (Family: none) | |
| JP 8-033077 A | 02.02.1996 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 4907991 B